(19) Europäisches Patentamt
European Patent Office
Office européen des brevets

(11) Veröffentlichungsnummer: **0 363 818 B1**

(12) # EUROPÄISCHE PATENTSCHRIFT

(45) Veröffentlichungstag der Patentschrift: **06.04.94**

(21) Anmeldenummer: **89118472.3**

(22) Anmeldetag: **05.10.89**

Die Akte enthält technische Angaben, die nach dem Eingang der Anmeldung eingereicht wurden und die nicht in dieser Patentschrift enthalten sind.

(51) Int. Cl.5: **C07D 213/70**, A01N 43/40, C07D 213/64, C07D 213/85, C07D 215/36, C07D 215/24, C07D 215/227, C07D 239/38, C07D 277/74, C07D 275/04, A01N 43/78

(54) **Oximether-Derivate, Verfahren zu ihrer Herstellung und diese enthaltende Fungizide.**

(30) Priorität: **14.10.88 DE 3835028**

(43) Veröffentlichungstag der Anmeldung:
**18.04.90 Patentblatt 90/16**

(45) Bekanntmachung des Hinweises auf die Patenterteilung:
**06.04.94 Patentblatt 94/14**

(84) Benannte Vertragsstaaten:
**AT BE CH DE ES FR GB GR IT LI NL SE**

(56) Entgegenhaltungen:
**EP-A- 0 253 213**
**EP-A- 0 254 426**
**EP-A- 0 278 595**
**EP-A- 0 299 694**

(73) Patentinhaber: **BASF Aktiengesellschaft**
**Carl-Bosch-Strasse 38**
**D-67063 Ludwigshafen(DE)**

(72) Erfinder: **Wenderoth, Bernd, Dr.**
**Schwalbenstrasse 26**
**D-6840 Lampertheim(DE)**
Erfinder: **Brand, Siegbert, Dr.**
**Hegelstrasse 39**
**D-6940 Weinheim(DE)**
Erfinder: **Schütz, Franz, Dr.**
**Budapester Strasse 45**
**D-6700 Ludwigshafen(DE)**
Erfinder: **Kuekenhoehner, Thomas, Dr.**
**Seidelstrasse 2**
**D-6710 Frankenthal(DE)**
Erfinder: **Roehl, Franz, Dr.**
**Karl-Otto-Braun-Strasse 8**
**D-6700 Ludwigshafen(DE)**
Erfinder: **Ammermann, Eberhard, Dr.**
**Sachsenstrasse 3**
**D-6700 Ludwigshafen(DE)**
Erfinder: **Lorenz, Gisela, Dr.**
**Erlenweg 13**
**D-6730 Neustadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 363 818 B1

## Beschreibung

Die vorliegende Erfindung betrifft neue Oximether-Derivate, ihre Herstellung, diese enthaltende Fungizide und ihre Verwendung als Fungizide.

Es ist bekannt, Oximether wie zum Beispiel das 2-(Phenyloxy- (oder Benzoxazolylthio)methyl)-phenylglyoxylsäuremethylester-O-methyloxim als Fungizide zu verwenden (EP-A-253 213, EP-A-254 426, EP-A-299 694). Es ist ferner bekannt, den α-(2-Pyridin-2-yloxymethyl)phenyl-β-methoxyacrylsäuremethylester als Fungizid zu verwenden (EP-A-278 595).

Es wurde nun gefunden, daß substituierte Oximether-Derivate der Formeln Ia und Ib

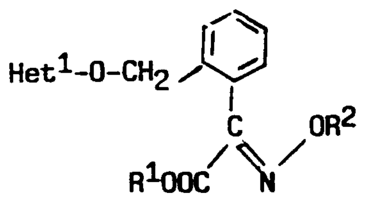

Ia

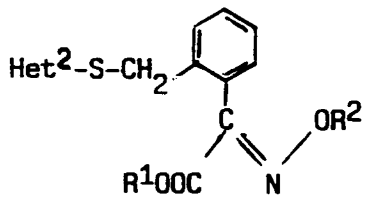

Ib

in der

| | |
|---|---|
| $R^1$ und $R^2$ | Wasserstoff oder $C_1$-$C_5$-Alkyl, |
| $Het^1$ | Pyridyl, Chinolyl, Pyrimidinyl, Pyrazinyl, Pyridazinyl, Thienyl, Chinoxalinyl, Isoxazolyl, Benzoxazolyl oder Benzthiazolyl bedeutet, wobei das heterocyclische Ringsystem gegebenenfalls substituiert ist durch Halogen, $C_1$-$C_8$-Alkyl, $C_3$-$C_6$-Cycloalkyl, $C_1$-$C_2$-Halogenalkyl, $C_1$-$C_4$-Alkoxy, $C_1$-$C_4$-Alkoxy-$C_1$-$C_4$-alkyl, Phenyl, Phenyl-$C_1$-$C_4$-alkyl, $C_1$-$C_4$-Alkylcarbonyl, $C_1$-$C_4$-Alkoxycarbonyl, Cyano, |
| $Het^2$ | 2-Pyridyl, 2-Chinolyl, 2-Pyrimidinyl bedeutet, wobei das heterocyclische Ringsystem gegebenenfalls substituiert ist durch Halogen, $C_1$-$C_8$-Alkyl, $C_3$-$C_6$-Cycloalkyl, $C_1$-$C_2$-Halogenalkyl, $C_1$-$C_4$-Alkoxy, $C_1$-$C_4$-Alkoxy-$C_1$-$C_4$-alkyl, Phenyl, Phenyl-$C_1$-$C_4$-alkyl, $C_1$-$C_4$-Alkylcarbonyl, $C_{1-4}$-Alkoxycarbonyl, Cyano, |

sowie ihre pflanzenverträglichen Säureadditionssalze, Metallkomplexverbindungen und ihre N-Oxide eine ausgezeichnete fungizide Wirkung besitzen.

Die in der allgemeinen Formel Ia und Ib aufgeführten Reste können beispielsweise folgende Bedeutung haben:

$R^1$ und $R^2$ sind gleich oder verschieden und stehen z. B. für Wasserstoff, $C_1$-$C_5$-Alkyl, Methyl, Ethyl, Propyl, i-Propyl, Butyl, Pentyl.

$Het^1$ kann z. B. 2-Pyridyl, 3-Pyridyl, 4-Pyridyl, 2-Chinolyl, 3-Chinolyl, 4-Chinolyl, 8-Chinolyl, 2-Pyrimidinyl, 4-Pyrimidinyl, 5-Pyrimidinyl, 2-Pyrazinyl, 3-Pyridazinyl, 2-Thienyl, 3-Thienyl, 2-Chinoxalinyl, 5-Isoxazolyl, 2-Benzoxazolyl oder 2-Benzthiazolyl sein.

Das heterocyclische Ringsystem $Het^1$ und $Het^2$ kann gegebenenfalls durch einen bis zu drei der folgenden Reste substituiert sein kann:
Halogen (z. B. Fluor, Chlor, Brom), $C_1$-$C_8$-Alkyl (z. B. Methyl, Ethyl, n- oder iso-Propyl, n-, iso-, sec- oder tert.-Butyl, n-, iso-, sec.-, tert.- oder neo-Pentyl, Hexyl, Heptyl, Octyl), $C_3$-$C_6$-Cycloalkyl (z. B. Cyclopropyl, Cyclobutyl, Cyclopentyl, Cyclohexyl), $C_1$-$C_2$-Halogenalkyl (z. B. Difluormethyl, Trifluormethyl, Chlormethyl, Dichlormethyl, Trichlormethyl, Pentafluorethyl), $C_1$-$C_4$-Alkoxy (z. B. Methoxy, Ethoxy, n-oder iso-Propoxy, n-, iso-, sec.- oder tert.-Butoxy), $C_1$-$C_4$-Alkoxy-$C_1$-$C_4$-alkyl (z. B. Methoxymethyl, Ethoxymethyl, Methoxyethyl, Propoxymethyl), Phenyl, Phenyl-$C_1$-$C_4$-alkyl (z. B. Benzyl, 2-Phenylethyl, 3-Phenylpropyl, 4-Phenylbutyl), $C_1$-$C_4$-Alkylcarbonyl (z. B. Acetyl, Ethylcarbonyl, Propylcarbonyl, Butylcarbonyl), $C_1$-$C_4$-Alkoxycarbonyl (z. B. Methoxycarbonyl, Ethoxycarbonyl, Propoxycarbonyl, Butoxycarbonyl), Cyano.

Die neuen Verbindungen können durch Umsetzung mit Säuren auch in pflanzenverträgliche Säureadditionssalze der anorganischen oder organischen Säuren überführt werden, wie beispielsweise in Salze der Salzsäure, Bromwasserstoffsäure, Salpetersäure, Oxalsäure, Essigsäure, Schwefelsäure, Phosphorsäure oder Dodecylbenzolsulfonsäure. Die Wirksamkeit der Salze geht auf das Kation zurück, so daß die Wahl des Anions im allgemeinen beliebig ist.

Ferner lassen sich die neuen Verbindungen nach bekannten Methoden in Metallkomplexe überführen. Das kann durch Umsetzung dieser Verbindungen mit Metallsalzen, z. B. der Metalle Kupfer, Zink, Eisen, Mangan oder Nickel, beispielsweise Kupfer(II)-chlorid, Zink(II)-chlorid, Eisen(III)-chlorid, Kupfer(II)-nitrat, Mangan(II)-chlorid oder Nickel(II)-bromid erfolgen.

Durch Umsetzung der neuen Verbindungen mit Oxidationsmitteln z. B. mit m-Chlorperbenzosäure erhält man die N-Oxide der heterocyclischen Verbindungen.

Die neuen Verbindungen der Formel Ia und Ib können bei ihrer Herstellung aufgrund der C=N-Doppelbindung als E/Z-Isomerengemische anfallen, die in üblicher Weise, z. B. durch Kristallisation oder Chromatographie, in die einzelnen Komponenten getrennt werden können. Sowohl die einzelnen isomeren Verbindungen als auch ihre Gemische werden von der Erfindung umfaßt und sind als Fungizide brauchbar.

Die neuen Verbindungen der allgemeinen Formel Ia und Ib lassen sich beispielsweise herstellen, indem man die heterocyclischen Verbindungen der Formeln $Het^1$-O-H (IIa) oder $Het^2$-S-H (IIb) mit einem Benzylhalogenid der allgemeinen Formel III umsetzt (Hal = Cl, Br, J). z.B.

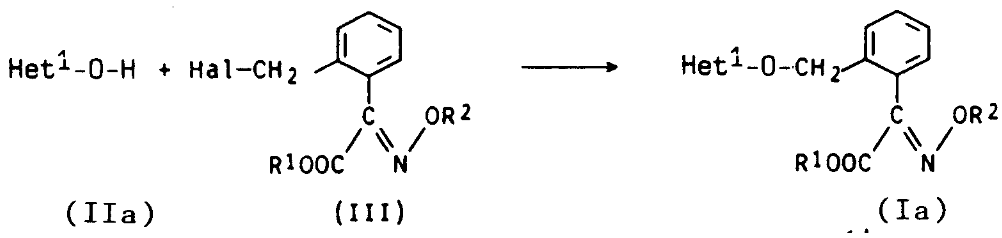

(IIa) (III) (Ia)

Die Umsetzungen zu den Verbindungen der Formel Ia und b können z. B. in einem inerten Lösungs- oder Verdünnungsmittel (z. B. Aceton, Acetonitril, Dimethylsulfoxid, Dioxan, Dimethylformamid, N-Methylpyrrolidon, N,N'-Dimethylpropylenharnstoff oder Pyridin) unter Verwendung einer Base (z. B. Natriumcarbonat, Kaliumcarbonat) durchgeführt werden. Außerdem kann es von Vorteil sein, der Reaktionsmischung einen Katalysator wie z. B. Tris-(3,6-dioxoheptyl)-amin zuzusetzen (J. Org. Chem. 50 (1985) 3717).

Alternativ kann auch so vorgegangen werden, daß man die Verbindungen der allgemeinen Formel IIa bzw. IIb zunächst mit einer Base (z. B. Natriumhydroxid oder Kaliumhydroxid) in die entsprechenden Natrium-oder Kaliumsalze überführt und diese dann in einem inerten Lösungs-oder Verdünnungsmittel (z. B. Dimethylformamid) mit den Benzylhalogeniden der Formel III zu den entsprechenden Verbindungen der allgemeinen Formel I umsetzt.

Die heterocyclischen Ausgangsverbindungen der allgemeinen Formel IIa und IIb sind entweder bekannt oder können durch Verfahren analog zu bekannten Verfahren hergestellt werden. Entsprechende Herstellverfahren sind z. B. beschrieben in EP-A-224 217; DE-A-25 31 035; J. Heterocyclic Chem. 20 (1983) 219; J. Heterocyclic Chem. 24 (1987) 709.

Die ortho-substituierten Benzylhalogenide der Formel III lassen sich herstellen, indem man literaturbekannte α-Ketocarbonsäureester der Formel IV (vgl. z. B. J. H. Photis, Tetrahedron Lett. 1980, 3539) nach literaturbekannten Methoden an der Methylgruppe halogeniert.

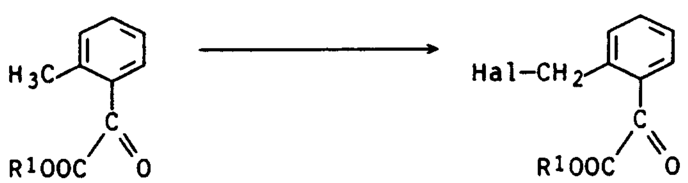

(IV) (V)

Mit Brom oder Chlor in einem Lösungsmittel wie zum Beispiel Tetrachlormethan, gegebenenfalls unter Belichtung mit einer Lichtquelle (zum Beispiel Hg-Dampf-Lampe, 300 W), mit N-Chlor- oder N-Bromsuccinimid (Horner, Winkelmann, Angew. Chem. 71, 349 (1959)) gelangt man z. B. zu den α-Ketocarbonsäureestern der allgemeinen Formel V, wobei $R^1$ die obengenannten Bedeutungen hat.

Die Halogenide der Formel III lassen sich herstellen, indem man einen α-Ketocarbonsäureester der Formel V z.B. a) mit O-substituierten Hydroxylaminen der Formel $H_2N$-$OR^2$ umsetzt, in der $R^2$ die obengenannten Bedeutungen hat, oder b) mit Hydroxylamin zum entsprechenden Oxim umsetzt und danach mit einem Alkylhalogenid der Formel $R^2$-Hal in der $R^2$ die obengenannten Bedeutungen hat und Hal ein Halogenatom (F, Cl, Br, J) bedeutet, oder mit einem Dialkylsulfat umsetzt.

Die Halogenide der Formel III lassen sich auch herstellen, indem man einen α-Ketocarbonsäureester der Formel IV z.B. a) mit O-substituierten Hydroxylaminen der Formel $H_2N$-$OR^2$ umsetzt, in der $R^2$ die obengenannten Bedeutungen hat, oder b) mit Hydroxylamin zum entsprechenden Oxim umsetzt und

danach mit einem Alkylhalogenid der Formel $R^2$-Hal in der $R^2$ die obengenannten Bedeutungen hat und Hal ein Halogenatom (F, Cl, Br, J) bedeutet, oder mit einem Dialkylsulfat zu Oximether-Derivaten der allgemeinen Formel VI umsetzt, wobei $R^1$ und $R^2$ die jeweils oben genannten Bedeutungen haben.

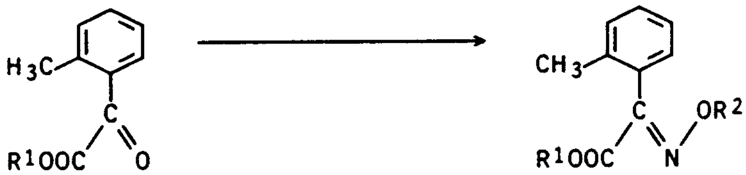

(IV) (VI)

Die Verbindungen der Formel VI können dann durch Halogenierung an der Methylgruppe nach literaturbekannten Methoden in die Halogenide der Formel III umgewandelt werden. Dies erreicht man zum Beispiel mit Brom oder Chlor in einem Lösungsmittel wie zum Beispiel Tetrachlormethan, gegebenenfalls unter Belichtung mit einer Lichtquelle (zum Beispiel Hg-Dampf-Lampe, 300 W), mit N-Chlor- oder N-Bromsuccinimid (Horner, Winkelmann, Angew. Chem. 71, 349 (1959)).

Die neuen Verbindungen der Formel I können zum Beispiel auch in der Weise hergestellt werden, daß man die neuen α-Ketocarbonsäureester der der Formel VII, worin Het-X = $Het^1$-O oder $Het^2$-S

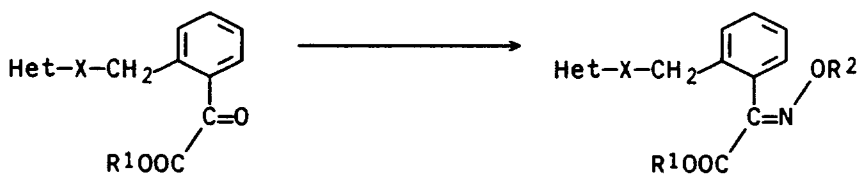

(VII) (I)

a) mit O-substituierten Hydroxylaminen der Formel $H_2NOR^2$ umsetzt, in der $R^2$ die obengenannten Bedeutungen hat,
oder
b) mit Hydroxylamin zum entsprechenden Oxim und danach mit einem Alkylhalogenid der Formel $R^2$-Hal, in der $R^2$ die obengenannten Bedeutungen hat und Hal ein Halogenatom (F, Cl, Br, J) bedeutet, oder mit einem Dialkylsulfat umsetzt.

Die neuen α-Ketocarbonsäureester der allgemeinen Formel VII, worin Het-X = $Het^1$-O oder $Het^2$-O, sind wertvolle Zwischenprodukte. Sie können zum Beispiel hergestellt werden, indem man die obengenannte Verbindung der Formel V mit den heterocyclischen Verbindungen der Formel II umsetzt.

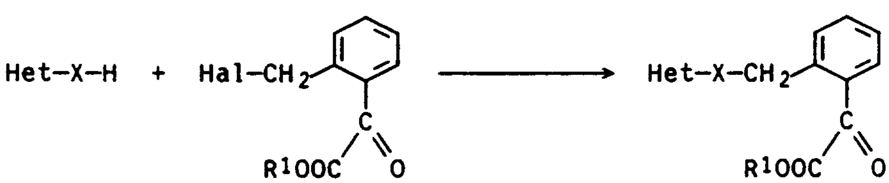

(II) (V) (VII)

Die Umsetzungen zu den Verbindungen der Formel VII können z. B. in einem inerten Lösungs- oder Verdünnungsmittel (z. B. Aceton, Acetonitril, Dimethylsulfoxid, Dioxan, Dimethylformamid, N-Methylpyrrolidon, N,N'-Dimethylpropylenharnstoff oder Pyridin) unter Verwendung einer Base (z. B. Natriumcarbonat, Kaliumcarbonat) durchgeführt werden. Außerdem kann es von Vorteil sein, der Reaktionsmischung einen Katalysator wie z. B. Tris-(3,6-dioxoheptyl)-amin zuzusetzen (J. Org. Chem. 50 (1985) 3717).

Alternativ kann auch so vorgegangen werden, daß man die Verbindungen der allgemeinen Formel II zunächst mit einer Base (z. B. Natriumhydroxid oder Kaliumhydroxid) in die entsprechenden Natrium-oder Kaliumsalze überführt und diese dann in einem inerten Lösungs-oder Verdünnungsmittel (z. B. Dimethylformamid) mit den Benzylhalogeniden der Formel V zu den neuen $\alpha$-Ketocarbonsäureestern der allgemeinen Formel VII umsetzt.

Beispiele

Die folgenden Beispiele sollen die Herstellung der neuen Wirkstoffe der allgemeinen Formel I erläutern.

Vorschrift 1

Herstellung von 2-(Brommethyl)-phenylglyoxylsäuremethylester

5,34 g (30 mmol) 2-Methylphenylglyoxylsäuremethylester und 5,34 g (30 mmol) N-Bromsuccinimid werden in 1000 l Tetrachlormethan für eine Stunde mit einer 300 W-Hg-Dampf-Lampe bestrahlt. Dann wird die organische Phase 1x mit Wasser und 3x mit Natriumhydrogencarbonat-Lösung gewaschen und über Natriumsulfat/Natriumcarbonat getrocknet. Nach dem Einengen wird das Rohprodukt an Kieselgel mit Methyl-t.-Butylether/n-Hexan (1/9) chromatographiert. Man erhält 3,8 g (49 %) der obengenannten Verbindungen als gelbes öl.
$^1$H-NMR ($CDCl_3$):
$\delta$ = 3,97 (s, 3H), 4,90 (s, 2H), 7,4-7,8 (m, 4H).
IR (Film):
2955, 1740, 1689, 1435, 1318, 1207, 999 $cm^{-1}$

Vorschrift 2

Herstellung von 2-(Chlormethyl)-phenylglyoxylsäuremethylester

100 g (0,56 mol) 2-Methylphenylglyoxylsäuremethylester werden in 600 ml Tetrachlormethan vorgelegt. Dann wird unter Bestrahlung mit einer 300 W-Hg-Dampf-Lampe auf Rückfluß erhitzt und dabei 28 g (0,39 mol) Chlorgas eingeleitet. Nach ca. 50 % Umsatz (DC-Kontrolle) wird die Reaktion abgebrochen und es wird eingeengt. Anschließend wird fraktioniert destilliert. Man erhält 29 g als öl.
$^1$H-NMR ($CDCl_3$):
$\delta$ = 3,97 (s, 3H), 5,0 (s, 2H), 7,4-7,8 (m, 4H).

Vorschrift 3

Herstellung von 2-(Brommethyl)phenylglyoxylsäuremethylester-O-methyloxim

27,5 g (0,133 mol) 2-Methyl-phenylglyoxylsäuremethylester-O-methyloxim gelöst in 400 ml Tetrachlormethan werden unter Rühren mit 21,4 g (0,133 mol) Brom versetzt. Dann wird unter Bestrahlung mit einer 300 W-Hg-Dampf-Lampe vier Stunden auf Rückflußtemperatur erhitzt. Danach wird eingeengt, in Essigester/Wasser aufgenommen, mit $H_2O$ gewaschen, mit Natriumsulfat getrocknet und eingeengt. Das Rohprodukt wird mit Cyclohexan/Essigester (9/1) an Kieselgel chromatographisch gereinigt. Es werden 17,4 g (46 %) der oben genannten Verbindung als öl erhalten.
$^1$H ($CDCl_3$):
$\delta$ = 3,88 /s, 3H), 4,08 (s, 3H), 4,33 (s, 2H), 6,12-7,52 (m, 4H).

Beispiel 1

Herstellung von 2-[(6'-Methyl-2'-pyridyl)-oxymethyl]-phenylglyoxylsäuremethyl-ester-O-meth yloxim (Verbindung Nr. 2)

4,85 g (37 mmol) 2-Hydroxy-6-methylpyridin-Natriumsalz werden zusammen mit 0,1 g Kaliumjodid in 100 ml absolutem N,N-Dimethylformamid vorgelegt. Dann werden unter Rühren bei Raumtemperatur (20 °C) 5,3 g (18,5 mmol) 2-(Brommethyl)-phenylglyoxylsäuremethylester-O-methyloxim, gelöst in 50 ml absolutem N,N-Dimethylformamid, zugetropft. Nachdem 5 Stunden bei 100 °C gerührt worden ist, wird eingeengt und der Rückstand in Essigester aufgenommen. Es wird mit $H_2O$ gewaschen, über Natriumsulfat getrocknet und eingeengt. Das Rohprodukt wird durch Verreiben mit n-Pentan zur Kristallisation gebracht. Die Kristalle werden isoliert und getrocknet. Man erhält 1,2 g (21 % Ausbeute) der oben genannten

Verbindung (Fp = 67-72 °C).
$^1$H-NMR ($CDCl_3$):
$\delta$ = 2,41 (s, 3H), 3,83 (s, 3H), 4,02 (s, 3H), 5,22 (s, 2H), 6,47 (d, 1H), 6,67 (d, 1H), 7,18-7,60 (m, 5H).

In entsprechender Weise können die in der folgenden Tabelle aufgeführten Verbindungen hergestellt werden.

Tabelle 1:

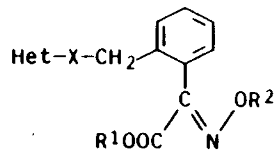

| Nr. | Het | Formel Ia oder Ib | $R^1$ | $R^2$ | Fp (°C) | IR ($cm^{-1}$) |
|---|---|---|---|---|---|---|
| 1 | 2-Pyridyl | b | $CH_3$ | $CH_3$ | | |
| 2 | 6-Methyl-2-pyridyl | a | $CH_3$ | $CH_3$ | 67-72 | 2940, 1742, 1601, 1580, 1456, 1305, 1227, 1070, 1101 |
| 3 | 6-Methyl-2-pyridyl | b | $CH_3$ | $CH_3$ | 104-108 | |
| 4 | 6-Ethyl-2-pyridyl | a | $CH_3$ | $CH_3$ | | |
| 5 | 6-Ethyl-2-pyridyl | b | $CH_3$ | $CH_3$ | 68-70 | |
| 6 | 6-n-Propyl-2-pyridyl | a | $CH_3$ | $CH_3$ | | |
| 7 | 6-n-Propyl-2-pyridyl | b | $CH_3$ | $CH_3$ | | |
| 8 | 6-iso-Propyl-2-pyridyl | a | $CH_3$ | $CH_3$ | 63-67 | 1731, 1459, 1441, 1069, 1011, 805, 777 |
| 9 | 6-iso-Propyl-2-pyridyl | b | $CH_3$ | $CH_3$ | 60-63 | |
| 10 | 6-n-Butyl-2-pyridyl | a | $CH_3$ | $CH_3$ | | |
| 11 | 6-n-Butyl-2-pyridyl | b | $CH_3$ | $CH_3$ | | |
| 12 | 6-tert.-Butyl-2-pyridyl | a | $CH_3$ | $CH_3$ | 73-75 | 1724, 1443, 1313, 1259, 1071, 1019, 803 |
| 13 | 6-tert.-Butyl-2-pyridyl | b | $CH_3$ | $CH_3$ | 98-103 | |
| 14 | 6-n-Pentyl-2-pyridyl | a | $CH_3$ | $CH_3$ | | |

Tabelle 1: (Fortsetzung)

| Nr. | Het | Formel Ia oder Ib | $R^1$ | $R^2$ | Fp (°C) | IR ($cm^{-1}$) |
|---|---|---|---|---|---|---|
| 15 | 6-n-Pentyl-2-pyridyl | b | $CH_3$ | $CH_3$ | | |
| 16 | 6-n-Hexyl-2-pyridyl | a | $CH_3$ | $CH_3$ | | |
| 17 | 6-n-Hexyl-2-pyridyl | b | $CH_3$ | $CH_3$ | | |
| 18 | 6-Phenyl-2-pyridyl | a | $CH_3$ | $CH_3$ | | |
| 19 | 6-Phenyl-2-pyridyl | b | $CH_3$ | $CH_3$ | | |
| 20 | 6-Benzyl-2-pyridyl | a | $CH_3$ | $CH_3$ | | |
| 21 | 6-Benzyl-2-pyridyl | b | $CH_3$ | $CH_3$ | | |
| 22 | 6-Trifluormethyl-2-pyridyl | a | $CH_3$ | $CH_3$ | | |
| 23 | 6-Trifluormethyl-2-pyridyl | b | $CH_3$ | $CH_3$ | | |
| 24 | 6-Methoxy-2-pyridyl | a | $CH_3$ | $CH_3$ | | |
| 25 | 6-Methoxy-2-pyridyl | b | $CH_3$ | $CH_3$ | | |
| 26 | 6-Chloro-2-pyridyl | a | $CH_3$ | $CH_3$ | | |
| 27 | 6-Chloro-2-pyridyl | b | $CH_3$ | $CH_3$ | 106 | 1740, 1592, 1442, 1032, 1059, 1009, 774 |
| 28 | 3,6-Dimethyl-2-pyridyl | a | $CH_3$ | $CH_3$ | | |
| 29 | 3,6-Dimethyl-2-pyridyl | b | $CH_3$ | $CH_3$ | | |
| 30 | 3,6-Diethyl-2-pyridyl | a | $CH_3$ | $CH_3$ | | |
| 31 | 3,6-Diethyl-2-pyridyl | b | $CH_3$ | $CH_3$ | | |
| 32 | 4,6-Dimethyl-2-pyridyl | a | $CH_3$ | $CH_3$ | | |
| 33 | 4,6-Dimethyl-2-pyridyl | b | $CH_3$ | $CH_3$ | | |
| 34 | 5,6-Dimethyl-2-pyridyl | a | $CH_3$ | $CH_3$ | | |
| 35 | 5,6-Dimethyl-2-pyridyl | b | $CH_3$ | $CH_3$ | | |
| 36 | 4-Phenyl-6-methyl-2-pyridyl | a | $CH_3$ | $CH_3$ | | |
| 37 | 4-Phenyl-6-methyl-2-pyridyl | b | $CH_3$ | $CH_3$ | | |

Tabelle 1: (Fortsetzung)

| Nr. | Het | Formel Ia oder Ib | $R^1$ | $R^2$ | Fp ($^oC$) | IR ($cm^{-1}$) |
|---|---|---|---|---|---|---|
| 38 | 4,6-Diphenyl-2-pyridyl | a | $CH_3$ | $CH_3$ | | |
| 39 | 4,6-Diphenyl-2-pyridyl | b | $CH_3$ | $CH_3$ | | |
| 40 | 3,4-Dichloro-6-methyl-2-pyridyl | a | $CH_3$ | $CH_3$ | | |
| 41 | 3,4-Dichloro-6-methyl-2-pyridyl | b | $CH_3$ | $CH_3$ | | |
| 42 | 3,4,5-Trichloro-6-phenyl-2-pyridyl | a | $CH_3$ | $CH_3$ | | |
| 43 | 3,4,5-Trichloro-6-phenyl-2-pyridyl | b | $CH_3$ | $CH_3$ | | |
| 44 | 4-Trifluoromethyl-6-methyl-2-pyridyl | a | $CH_3$ | $CH_3$ | | |
| 45 | 4-Trifluoromethyl-6-methyl-2-pyridyl | b | $CH_3$ | $CH_3$ | | |
| 46 | 3-Acetyl-4,6-dimethyl-2-pyridyl | a | $CH_3$ | $CH_3$ | | |
| 47 | 3-Acetyl-4,6-dimethyl-2-pyridyl | b | $CH_3$ | $CH_3$ | | |
| 48 | 3-Cyano-6-methyl-2-pyridyl | a | $CH_3$ | $CH_3$ | 132-135 | 2215, 1729, 1594, 1061, 1013, 760 |
| 49 | 3-Cyano-6-methyl-2-pyridyl | b | $CH_3$ | $CH_3$ | | |
| 50 | 3-Cyano-6-ethyl-2-pyridyl | a | $CH_3$ | $CH_3$ | 87-90 | |
| 51 | 3-Cyano-6-ethyl-2-pyridyl | b | $CH_3$ | $CH_3$ | | |
| 52 | 3-Cyano-6-n-propyl-2-pyridyl | a | $CH_3$ | $CH_3$ | | |
| 53 | 3-Cyano-6-n-propyl-2-pyridyl | b | $CH_3$ | $CH_3$ | | |
| 54 | 3-Cyano-6-iso-propyl-2-pyridyl | a | $CH_3$ | $CH_3$ | | |
| 55 | 3-Cyano-6-iso-propyl-2-pyridyl | b | $CH_3$ | $CH_3$ | | |
| 56 | 3-Cyano-6-cyclo-propyl-2-pyridyl | a | $CH_3$ | $CH_3$ | | |
| 57 | 3-Cyano-6-cyclo-propyl-2-pyridyl | b | $CH_3$ | $CH_3$ | | |
| 58 | 3-Cyano-6-n-butyl-2-pyridyl | a | $CH_3$ | $CH_3$ | | |
| 59 | 3-Cyano-6-n-butyl-2-pyridyl | b | $CH_3$ | $CH_3$ | | |
| 60 | 3-Cyano-6-tert.-butyl-2-pyridyl | a | $CH_3$ | $CH_3$ | | |

Tabelle 1: (Fortsetzung)

| Nr. | Het | Formel Ia oder Ib | $R^1$ | $R^2$ | Fp (°C) | IR ($cm^{-1}$) |
|---|---|---|---|---|---|---|
| 61 | 3-Cyano-6-tert.-butyl-2-pyridyl | b | $CH_3$ | $CH_3$ | | |
| 62 | 3-Cyano-6-cyclo-hexyl-2-pyridyl | a | $CH_3$ | $CH_3$ | | |
| 63 | 3-Cyano-6-cyclo-hexyl-2-pyridyl | b | $CH_3$ | $CH_3$ | | |
| 64 | 3-Cyano-6-phenyl-2-pyridyl | a | $CH_3$ | $CH_3$ | 123-125 | 2315, 1727, 1591, 1456, 1220, 1065, 1019, 771 753 |
| 65 | 3-Cyano-6-phenyl-2-pyridyl | b | $CH_3$ | $CH_3$ | | |
| 66 | 3-Methyloxycarbonyl-6-iso-propyl-2-pyridyl | a | $CH_3$ | $CH_3$ | | |
| 67 | 3-Methyloxycarbonyl-6-iso-propyl-2-pyridyl | b | $CH_3$ | $CH_3$ | | |
| 68 | 3-Ethyloxycarbonyl-6-iso-propyl-2-pyridyl | a | $CH_3$ | $CH_3$ | | |
| 69 | 3-Ethyloxycarbonyl-6-iso-propyl-2-pyridyl | b | $CH_3$ | $CH_3$ | | |
| 70 | 3-Cyano-4,6-dimethyl-2-pyridyl | a | $CH_3$ | $CH_3$ | | |
| 71 | 3-Cyano-4,6-dimethyl-2-pyridyl | b | $CH_3$ | $CH_3$ | | |
| 72 | 3,5,6-Trichloro-2-pyridyl | a | $CH_3$ | $CH_3$ | | |
| 73 | 3,5,6-Trichloro-2-pyridyl | b | $CH_3$ | $CH_3$ | | |
| 74 | 5-Trifluoromethyl-2-pyridyl | a | $CH_3$ | $CH_3$ | | |
| 75 | 5-Trifluoromethyl-2-pyridyl | b | $CH_3$ | $CH_3$ | | |
| 76 | 3-Chloro-5-trifluoromethyl-2-pyridyl | a | $CH_3$ | $CH_3$ | | |
| 77 | 3-Chloro-5-trifluoromethyl-2-pyridyl | b | $CH_3$ | $CH_3$ | | |
| 78 | 2-Chinolyl | a | $CH_3$ | $CH_3$ | | |
| 79 | 2-Chinolyl | b | $CH_3$ | $CH_3$ | 103-108 | 1725, 1593, 1435, 1213, 1090, 1067, 1021, 822, 759 |
| 80 | 3-Methyl-2-chinolyl | a | $CH_3$ | $CH_3$ | | |
| 81 | 3-Methyl-2-chinolyl | b | $CH_3$ | $CH_3$ | | |
| 82 | 4-Methyl-2-chinolyl | a | $CH_3$ | $CH_3$ | 137-139 | 1722, 1605, 1337, 1184, 1011, 757 |

Tabelle 1: (Fortsetzung)

| Nr. | Het | Formel Ia oder Ib | $R^1$ | $R^2$ | Fp (°C) | IR ($cm^{-1}$) |
|---|---|---|---|---|---|---|
| 83 | 4-Methyl-2-chinolyl | b | $CH_3$ | $CH_3$ | | |
| 84 | 4-Ethyl-2-chinolyl | a | $CH_3$ | $CH_3$ | | |
| 85 | 4-Ethyl-2-chinolyl | b | $CH_3$ | $CH_3$ | | |
| 86 | 4-Phenyl-2-chinolyl | a | $CH_3$ | $CH_3$ | | |
| 87 | 4-Phenyl-2-chinolyl | b | $CH_3$ | $CH_3$ | | |
| 88 | 6-Methyl-2-chinolyl | a | $CH_3$ | $CH_3$ | | |
| 89 | 6-Methyl-2-chinolyl | b | $CH_3$ | $CH_3$ | | |
| 90 | 6-Chloro-2-chinolyl | a | $CH_3$ | $CH_3$ | | |
| 91 | 6-Chloro-2-chinolyl | b | $CH_3$ | $CH_3$ | | |
| 92 | 8-Methyl-2-chinolyl | a | $CH_3$ | $CH_3$ | | |
| 93 | 8-Methyl-2-chinolyl | b | $CH_3$ | $CH_3$ | | |
| 94 | 8-Chloro-2-chinolyl | a | $CH_3$ | $CH_3$ | | |
| 95 | 8-Chloro-2-chinolyl | b | $CH_3$ | $CH_3$ | | |
| 96 | 4-Ethoxycarbonyl-2-chinolyl | a | $CH_3$ | $CH_3$ | | |
| 97 | 4-Ethoxycarbonyl-2-chinolyl | b | $CH_3$ | $CH_3$ | | |
| 98 | 3,4-Dimethyl-2-chinolyl | a | $CH_3$ | $CH_3$ | | |
| 99 | 3,4-Dimethyl-2-chinolyl | b | $CH_3$ | $CH_3$ | | |
| 100 | 4-Methyl-8-methoxy-2-chinolyl | a | $CH_3$ | $CH_3$ | | |
| 101 | 4-Methyl-8-methoxy-2-chinolyl | b | $CH_3$ | $CH_3$ | | |
| 102 | 4-Phenyl-8-ethoxy-2-chinolyl | a | $CH_3$ | $CH_3$ | | |
| 103 | 4-Phenyl-8-ethoxy-2-chinolyl | b | $CH_3$ | $CH_3$ | | |
| 104 | 4-Methyl-8-chloro-2-chinolyl | a | $CH_3$ | $CH_3$ | | |
| 105 | 4-Methyl-8-chloro-2-chinolyl | b | $CH_3$ | $CH_3$ | | |

Tabelle 1: (Fortsetzung)

| Nr. | Het | Formel Ia oder Ib | $R^1$ | $R^2$ | Fp (°C) | IR ($cm^{-1}$) |
|---|---|---|---|---|---|---|
| 106 | 4-Methyl-8-fluoro-2-chinolyl | a | $CH_3$ | $CH_3$ | | |
| 107 | 4-Methyl-8-fluoro-2-chinolyl | b | $CH_3$ | $CH_3$ | | |
| 108 | 4-Chinolyl | a | $CH_3$ | $CH_3$ | | |
| 109 | 2-Methyl-4-chinolyl | a | $CH_3$ | $CH_3$ | | |
| 110 | 2-Trichloromethyl-4-chinolyl | a | $CH_3$ | $CH_3$ | | |
| 111 | 2-Trifluoromethyl-2-chinolyl | a | $CH_3$ | $CH_3$ | | |
| 112 | 2-iso-Propyl-4-chinolyl | a | $CH_3$ | $CH_3$ | | |
| 113 | 2-n-Pentyl-4-chinolyl | a | $CH_3$ | $CH_3$ | | |
| 114 | 2-Phenyl-4-chinolyl | a | $CH_3$ | $CH_3$ | | |
| 115 | 2-Methoxycarbonyl-4-chinolyl | a | $CH_3$ | $CH_3$ | | |
| 116 | 2,6-Dimethyl-4-chinolyl | a | $CH_3$ | $CH_3$ | | |
| 117 | 2-Methyl-6-chloro-4-chinolyl | a | $CH_3$ | $CH_3$ | | |
| 118 | 2-Methyl-6-fluoro-4-chinolyl | a | $CH_3$ | $CH_3$ | | |

Tabelle 1: (Fortsetzung)

| Nr. | Het | Formel Ia oder Ib | $R^1$ | $R^2$ | Fp (°C) | IR ($cm^{-1}$) |
|---|---|---|---|---|---|---|
| 130 | 8-Chinolyl | a | $CH_3$ | $CH_3$ | | |
| 132 | 2-Methyl-8-chinolyl | a | $CH_3$ | $CH_3$ | | |
| 119 | 5,7-Dichloro-8-chinolyl | a | $CH_3$ | $CH_3$ | 120-122 | 1730, 1062, 1009, 793 742 |
| 120 | 4,6-Dimethyl-2-pyrimidinyl | a | $CH_3$ | $CH_3$ | | |
| 121 | 4,6-Dimethyl-2-pyrimidinyl | b | $CH_3$ | $CH_3$ | | |
| 122 | 4-Trifluormethyl-2-pyrimidinyl | a | $CH_3$ | $CH_3$ | | |
| 123 | 4-Trifluormethyl-2-pyrimidinyl | b | $CH_3$ | $CH_3$ | | |
| 124 | 4,5,6-Trimethyl-2-pyrimidinyl | a | $CH_3$ | $CH_3$ | | |
| 125 | 4,5,6-Trimethyl-2-pyrimidinyl | b | $CH_3$ | $CH_3$ | | |
| 126 | 4-Benzyl-6-methyl-2-pyrimidinyl | a | $CH_3$ | $CH_3$ | | |
| 127 | 4-Benzyl-6-methyl-2-pyrimidinyl | b | $CH_3$ | $CH_3$ | | |
| 128 | 4-Methyl-6-phenyl-2-pyrimidinyl | a | $CH_3$ | $CH_3$ | | |
| 129 | 4-Methyl-6-phenyl-2-pyrimidinyl | b | $CH_3$ | $CH_3$ | | |
| 130 | 4,6-Dimethyl-5-chloro-2-pyrimidinyl | a | $CH_3$ | $CH_3$ | | |
| 131 | 4,6-Dimethyl-5-chloro-2-pyrimidinyl | b | $CH_3$ | $CH_3$ | | |
| 132 | 2,6-Dimethyl-4-pyrimidinyl | a | $CH_3$ | $CH_3$ | | |
| 133 | 2,6-Bis-(trifluormethyl)-4-pyrimidinyl | a | $CH_3$ | $CH_3$ | | |

Tabelle 1: (Fortsetzung)

| Nr. | Het | Formel Ia oder Ib | $R^1$ | $R^2$ | Fp (°C) | IR ($cm^{-1}$) |
|---|---|---|---|---|---|---|
| 134 | 2-Chloromethyl-6-metyhl-4-pyrimidinyl | a | $CH_3$ | $CH_3$ | | |
| 135 | 2-Methyl-6-chloromethyl-4-pyrimidinyl | a | $CH_3$ | $CH_3$ | | |
| 136 | 2-iso-Propyl-6-methyl-4-pyrimidinyl | a | $CH_3$ | $CH_3$ | | |
| 137 | 2-iso-Propyl-6-chloromethyl-4-pyrimidinyl | a | $CH_3$ | $CH_3$ | | |
| 138 | 2-cyclo-Propyl-6-chloromethyl-4-pyrimidinyl | a | $CH_3$ | $CH_3$ | | |
| 139 | 2-cyclo-Propyl-6-methyl-4-pyrimidinyl | a | $CH_3$ | $CH_3$ | | |
| 140 | 2-Methyl-6-methoxymethyl-4-pyrimidinyl | a | $CH_3$ | $CH_3$ | | |
| 141 | 2-iso-Propyl-6-methoxymethyl-4-pyrimidinyl | a | $CH_3$ | $CH_3$ | | |
| 142 | 2-Phenyl-4-pyrimidinyl | a | $CH_3$ | $CH_3$ | | |
| 143 | 3,5-Dimethyl-4-pyrimidinyl | a | $CH_3$ | $CH_3$ | | |
| 144 | 2-Methylthio-6-trifluoromethyl-4-pyrimidinyl | a | $CH_3$ | $CH_3$ | | |
| 145 | 2-Methylthio-5-chloro-6-trifluoromethyl-4-pyrimidinyl | a | $CH_3$ | $CH_3$ | | |

Tabelle 1: (Fortsetzung)

| Nr. | Het | Formel Ia oder Ib | $R^1$ | $R^2$ | Fp (°C) | IR ($cm^{-1}$) |
|---|---|---|---|---|---|---|
| 146 | 2-Methylthio-5-n-octyl-6-methyl-4-pyrimidinyl | a | $CH_3$ | $CH_3$ | | |
| 147 | 2-Methyl-6-trifluoromethyl-4-pyrimidinyl | a | $CH_3$ | $CH_3$ | | |
| 148 | 2-n-Propyl-6-trifluoromethyl-4-pyrimidinyl | a | $CH_3$ | $CH_3$ | | |
| 149 | 2-iso-Propyl-6-trifluoromethyl-4-pyrimidinyl | a | $CH_3$ | $CH_3$ | | |
| 150 | 2-tert.-Butyl-6-trifluoromethyl-4-pyrimidinyl | a | $CH_3$ | $CH_3$ | | |
| 151 | 2-Methyl-5-chloro-6-trifluoromethyl-4-pyrimidinyl | a | $CH_3$ | $CH_3$ | | |
| 152 | 2-n-Propyl-5-chloro-6-trifluoromethyl-4-pyrimidinyl | a | $CH_3$ | $CH_3$ | | |
| 153 | 2-iso-Propyl-5-chloro-6-trifluoromethyl-4-pyrimidinyl | a | $CH_3$ | $CH_3$ | | |

Tabelle 1: (Fortsetzung)

| Nr. | Het | Formel Ia oder Ib | $R^1$ | $R^2$ | Fp (°C) | IR ($cm^{-1}$) |
|---|---|---|---|---|---|---|
| 154 | 2-tert.-Butyl-5-chloro-6-trifluoromethyl-4-pyrimidinyl | a | $CH_3$ | $CH_3$ | | |
| 155 | 2-Pyrimidinyl | b | $CH_3$ | $CH_3$ | | |
| 156 | 6-Methyl-2-pyridyl | a | H | $CH_3$ | | |
| 157 | 6-Methyl-2-pyridyl | a | $CH_3$ | H | | |
| 158 | 6-Methyl-2-pyridyl | b | $CH_3$ | H | | |
| 159 | 6-Methyl-2-pyridyl | a | $C_2H_5$ | $C_2H_5$ | | |
| 160 | 6-Methyl-2-pyridyl | b | $C_2H_5$ | $C_2H_5$ | | |
| 161 | 6-Methyl-2-pyridyl | a | $CH_3$ | $C_2H_5$ | | |
| 162 | 6-Methyl-2-pyridyl | b | $CH_3$ | $C_2H_5$ | | |
| 163 | 6-Methyl-2-pyridyl | a | $CH_3$ | $n-C_3H_7$ | | |
| 164 | 6-Methyl-2-pyridyl | a | $CH_3$ | $i-C_3H_7$ | | |
| 165 | 6-Methyl-2-pyridyl | a | $CH_3$ | $C_4H_9$ | | |
| 166 | 6-Methyl-2-pyridyl | a | $CH_3$ | $C_5H_{11}$ | | |
| 167 | 6-Cyclopropyl-2-pyridyl | a | $CH_3$ | $CH_3$ | | |
| 168 | 6-Cyclopropyl-2-pyridyl | b | $CH_3$ | $CH_3$ | | |
| 169 | 2-Pyrazinyl | a | $CH_3$ | $CH_3$ | | |
| 170 | 6-Chlor-2-pyrazinyl | a | $CH_3$ | $CH_3$ | | |
| 171 | 5-Methyl-2-pyrazinyl | a | $CH_3$ | $CH_3$ | | |

Tabelle 1: (Fortsetzung)

| Nr. | Het | Formel Ia oder Ib | $R^1$ | $R^2$ | Fp (°C) | IR ($cm^{-1}$) |
|---|---|---|---|---|---|---|
| 172 | 3-Pyridazinyl | a | $CH_3$ | $CH_3$ | | |
| 173 | 5-Chlor-3-pyridazinyl | a | $CH_3$ | $CH_3$ | | |
| 174 | 2-Thienyl | a | $CH_3$ | $CH_3$ | | |
| 175 | 3-Thienyl | a | $CH_3$ | $CH_3$ | | |
| 176 | 4-Chlor-3-thienyl | a | $CH_3$ | $CH_3$ | | |
| 177 | 2-Chlor-3-thienyl | a | $CH_3$ | $CH_3$ | | |
| 178 | 5-Chlor-3-thienyl | a | $CH_3$ | $CH_3$ | | |
| 179 | 2-Chinoxalinyl | a | $CH_3$ | $CH_3$ | | |
| 180 | 3-Methyl-2-chinxalinyl | a | $CH_3$ | $CH_3$ | | |
| 181 | 7,8-Dimethyl-2-chinoxalinyl | a | $CH_3$ | $CH_3$ | | |
| 182 | 7,8-Dichlor-2-chinoxalinyl | a | $CH_3$ | $CH_3$ | | |

Tabelle 1: (Fortsetzung)

| Nr. | Het | Formel Ia oder Ib | $R^1$ | $R^2$ | Fp (°C) | IR ($cm^{-1}$) |
|---|---|---|---|---|---|---|
| 236 | 7-Methyl-2-chinoxalinyl | a | $CH_3$ | $CH_3$ | | |
| 238 | 8-Methyl-2-chinoxalinyl | a | $CH_3$ | $CH_3$ | | |
| 183 | 7-Methoxy-2-chinoxalinyl | a | $CH_3$ | $CH_3$ | | |
| 242 | 3-Phenyl-5-isoxazolyl | a | $CH_3$ | $CH_3$ | | |
| 184 | 2-Benzoxazolyl | a | $CH_3$ | $CH_3$ | | |
| 185 | 2-Benzthiazolyl | a | $CH_3$ | $CH_3$ | | |
| 186 | 1,2-Benzthiazol-7-yl | a | $CH_3$ | $CH_3$ | 120-123 | |
| 187 | 4,8-Dimethyl-2-Chinolyl | b | $CH_3$ | $CH_3$ | 108-110 | |
| 188 | 4,8-Dimethyl-2-Chinolyl | a | $CH_3$ | $CH_3$ | 109-112 | |
| 189 | 6-iso-buyl-2-pyridyl | a | $CH_3$ | $CH_3$ | 84-86 | |
| 190 | 3-Cyano-6-iso-butyl-2-pyridyl | a | $CH_3$ | $CH_3$ | 83-86 | |
| 191 | 2-Pyrimidinyl | b | $CH_3$ | $CH_3$ | 96-98 | 1727, 1551, 1378, 1217, 1200, 1069, 1020, 756 |

Die neuen Verbindungen zeichnen sich, allgemein ausgedrückt, durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt- und Bodenfungizide eingesetzt werden.

Besonders interessant sind die fungiziden Verbindungen für die Bekämpfung einer Vielzahl von Pilzen an verschiedenen Kulturpflanzen oder ihren Samen, insbesondere Weizen, Roggen, Gerste, Hafer, Reis,

Mais, Rasen, Baumwolle, Soja, Kaffee, Zuckerrohr, Obst und Zierpflanzen im Gartenbau, Weinbau sowie Gemüse - wie Gurken, Bohnen und Kürbisgewächse -.

Die neuen Verbindungen sind insbesondere geeignet zur Bekämpfung folgender Pflanzenkrankheiten:

Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die Verbindungen werden angewendet, indem man die Pflanzen mit den Wirkstoffen besprüht oder bestäubt oder die Samen der Pflanzen mit den Wirkstoffen behandelt. Die Anwendung erfolgt vor oder nach der Infektion der Pflanzen oder Samen durch die Pilze.

Die neuen Substanzen können in die üblichen Formulierungen übergeführt werden, wie Lösungen, Emulsionen, Suspensionen, Stäube, Pulver, Pasten und Granulate. Die Anwendungsformen richten sich ganz nach den Verwendungszwecken; sie sollen in jedem Fall eine feine und gleichmäßige Verteilung der wirksamen Substanz gewährleisten. Die Formulierungen werden in bekannter Weise hergestellt, z.B. durch Verstrecken des Wirkstoffs mit Lösungsmitteln und/oder Trägerstoffen, gegebenenfalls unter Verwendung von Emulgiermitteln und Dispergiermitteln, wobei im Falle der Benutzung von Wasser als Verdünnungsmittel auch andere organische Lösungsmittel als Hilfslösungsmittel verwendet werden können. Als Hilfsstoffe kommen dafür im wesentlichen in Frage: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin, Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate) ; Emulgiermittel, wie nichtionogene und anionische Emulgatoren (z.B. Polyoxyethylen-Fettalkohol-Ether, Alkylsulfonate und Arylsulfonate) und Dispergiermittel, wie Lignin, Sulfitablaugen und Methylcellulose.

Die fungiziden Mittel enthalten im allgemeinen zwischen 0,1 und 95, vorzugsweise zwischen 0,5 und 90 Gew.% Wirkstoff.

Die Aufwandmengen liegen je nach Art des gewünschten Effektes zwischen 0,02 und 3 kg Wirkstoff oder mehr je ha. Die neuen Verbindungen können auch im Materialschutz eingesetzt werden, z.B. gegen Paecilomyces variotii.

Die Mittel bzw. die daraus hergestellten gebrauchsfertigen Zubereitungen, wie Lösungen, Emulsionen, Suspensionen, Pulver, Stäube, Pasten oder Granulate werden in bekannter Weise angewendet, beispielsweiise durch Versprühen, Vernebeln, Verstäuben, Verstreuen, Beizen oder Gießen.

Beispiele für solche Zubereitungen sind:

I. Man vermischt 90 Gew.-Teile der Verbindung Nr. 2 mit 10 Gew.-Teilen N-Methyl-$\alpha$-pyrrolidon und erhält eine Lösung, die zur Anwendung in Form kleinster Tropfen geeignet ist.

II. 20 Gew.-Teile der Verbindung Nr. 5 werden in einer Mischung gelöst, die aus 80 Gew.-Teilen Xylol, 10 Gew.-Teilen des Anlagerungsproduktes von 8 bis 10 Mol Ethylenoxid an 1 Mol ölsäure-N-monoethanolamid, 5 Gew.-Teilen Calciumsalz der Dodecylbenzolsulfonsäure und 5 Gew.-Teilen des Anlagerungsproduktes und 40 Mol Ethylenoxid an 1 Mol Ricinusöl besteht. Durch Ausgießen und feines Verteilen der Lösung in Wasser erhält man eine wäßrige Dispersion.

III. 20 Gew.-Teile der Verbindung Nr. 8 werden in einer Mischung gelöst, die aus 40 Gew.-Teilen Cyclohexanon, 30 Gew.-Teilen Isobutanol, 20 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 20 Gew.-Teile der Verbindung Nr. 12 werden in einer Mischung gelöst, die aus 25 Gew.-Teilen Cyclohexanol, 65 Gew.-Teilen einer Mineralölfraktion vom Siedepunkt 210 bis 280 °C und 10 Gew.-Teilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

V. 80 Gew.-Teile der Verbindung Nr. 2 werden mit 3 Gew.-Teilen des Natriumsalzes der Diisobutylnaphthalin-α-sulfonsäure, 10 Gew.-Teilen des Natriumsalzes einer Ligninsulfonsäure aus einer Sulfitablauge und 7 Gew.-Teilen pulverförmigem Kieselsäuregel gut vermischt und in einer Hammermühle vermahlen. Durch feines Verteilen der Mischung in Wasser erhält man eine Spritzbrühe.

VI. 3 Gew.-Teile der Verbindung Nr. 5 werden mit 97 Gew.-Teilen feinteiligem Kaolin innig vermischt. Man erhält auf diese Weise ein Stäubemittel, das 3 Gew.% des Wirkstoffs enthält.

VII. 30 Gew.-Teile der Verbindung Nr. 8 werden mit einer Mischung aus 92 Gew.-Teilen pulverförmigem Kieselsäuregel und 8 Gew.-Teilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VIII. 40 Gew.-Teile der Verbindung Nr. 12 werden mit 10 Gew.-Teilen Natriumsalz eines Phenolsulfonsäure-harnstoff-formaldehyd-KondenX sates, 2 Gew.-Teilen Kieselgel und 48 Gew.-Teilen Wasser innig vermischt. Man erhält eine stabile wäßrige Dispersion. Durch Verdünnen mit Wasser erhält man eine wäßrige Dispersion.

IX. 20 Gew.-Teile der Verbindung Nr. 2 werden mit 2 Gew.-Teilen Calciumsalz der Dodecylbenzolsulfonsäure, 8 Gew.-Teilen Fettalkoholpolyglykolether, 2 Gew.-Teilen Natriumsalz eines Phenolsulfonsäure-harnstoff-formaldehyd-Kondensats und 68 Gew.-Teilen eines paraffinischen Mineralöls innig vermischt. Man erhält eine stabile ölige Dispersion.

Die erfindungsgemäßen Mittel können in diesen Anwendungsformen auch zusammen mit anderen Wirkstoffen vorliegen, wie z.B. Herbiziden, Insektiziden, Wachstumsregulatoren und Fungiziden, oder auch mit Düngemitteln vermischt und ausgebracht werden. Beim Vermischen mit Fungiziden erhält man dabei in vielen Fällen eine Vergrößerung des fungiziden Wirkungsspektrums.

Anwendungsbeispiele

Als Vergleichswirkstoff wurde 2-(Phenyloxymethyl)-phenylglyoxylsäuremethylester-O-methyloXim (A) - bekannt aus EP-A-253 213 - benutzt.

Anwendungsbeispiel 1

Wirksamkeit gegen Rebenperonospora

Blätter von Topfreben der Sorte "Müller Thurgau" wurden mit wäßriger Spritzbrühe, die 80 % Wirkstoff und 20 % Emulgiermittel in der Trockensubstanz enthielt, besprüht. Um die Wirkungsdauer der Wirkstoffe beurteilen zu können, wurden die Pflanzen nach dem Antrocknen des Spritzbelages 8 Tage im Gewächshaus aufgestellt. Erst dann wurden die Blätter mit einer Zoosporenaufschwemmung von Plasmopara viticola (Rebenperonospora) infiziert. Danach wurden die Reben zunächst für 48 Stunden in einer wasserdampfgesättigten Kammer bei 24 °C und anschließend für 5 Tage in einem Gewächshaus mit Temperaturen zwischen 20 und 30 °C aufgestellt. Nach dieser Zeit wurden die Pflanzen zur Beschleunigung des sporangienträgerausbruches abermals für 16 Stunden in der feuchten Kammer aufgestellt. Dann erfolgte die Beurteilung des Ausmaßes des Pilzausbruches auf den Blattunterseiten.

Das Ergebnis zeigt, daß die Wirkstoffe Nr. 2, 3, 5, 8, 9, 12, 27, 50, und 189 bei der Anwendung als 0,05; 0,025 und 0,0125 %ige (Gew.-%) Spritzbrühen eine bessere fungizide Wirkung zeigen (100 %) als der bekannte Vergleichswirkstoff A (90 %).

Anwendungsbeispiel 2

Wirksamkeit gegen Phytophthora infestans

Blätter von Topfpflanzen der Sorte "Große Fleischtomate" wurden mit wäßriger Spritzbrühe, die 80 % Wirkstoff und 20 % Emulgiermittel in der Trockensubstanz enthielt, besprüht. Nach 24 Stunden wurden die Blätter mit einer Zoosporenaufschwemmung des Pilzes Phytophthora infestans infiziert. Die Pflanzen wurden dann in einer wasserdampfgesättigten Kammer bei Temperaturen zwischen 16 und 18 °C aufgestellt. Nach 6 Tagen hatte sich die Krankheit auf den unbehandelten, jedoch infizierten Kontrollpflanzen so

stark entwickelt, daß die fungizide Wirksamkeit der Substanzen beurteilt werden konnte.

Das Ergebnis zeigt, daß der Wirkstoff Nr. 2 bei der Anwendung als 0,025 %ige Spritzbrühe eine bessere fungizide Wirkung zeigt (90 %) als der bekannte Vergleichswirkstoff A (55 %).

Anwendungsbeispiel 3

Wirksamkeit gegen Pyrenophora teres

Gerstenkeimlinge der Sorte "Igri" wurden im Zweiblattstadium mit wäßrigen Suspensionen, die 80 % Wirkstoff und 20 % Emulgator in der Trockensubstanz enthielten, tropfnaß gespritzt. Nach 24 Stunden wurden die Pflanzen mit einer Sporensuspension des Pilzes Pyrenophora teres inokuliert und für 48 Stunden in eine Klimakammer mit hoher Luftfeuchtigkeit bei 18 °C gestellt. Anschließend wurden die Pflanzen im Gewächshaus bei 20 - 22 °C und 70 % relativer Luftfeuchtigkeit für weitere 5 Tage kultiviert. Dann wurde das Ausmaß des Befalls der Blätter ermittelt.

Das Ergebnis zeigt, daß die Wirkstofe Nr. 5, 8, 12, 13, 50 bei der Anwendung als 0,05 %ige Spritzbrühe eine bessere fungizide Wirkung zeigen (97 %) als der bekannte Vergleichswirkstoff A (55 %).

**Patentansprüche**

**1.** Oximether-Derivate der Formeln Ia und Ib

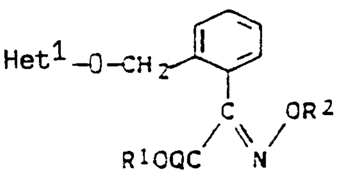 Ia 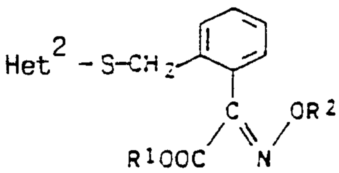 Ib

in der

$R^1$ und $R^2$ Wasserstoff oder $C_1$-$C_5$-Alkyl,

$Het^1$ Pyridyl, Chinolyl, Pyrimidinyl, Pyrazinyl, Pyridazinyl, Thienyl, Chinoxalinyl, Isoxazolyl, Benzoxazolyl oder Benzthiazolyl bedeutet, wobei das heterocyclische Ringsystem gegebenenfalls substituiert ist durch Halogen, $C_1$-$C_8$-Alkyl, $C_3$-$C_6$-Cycloalkyl, $C_1$-$C_2$-Halogenalkyl, $C_1$-$C_4$-Alkoxy, $C_1$-$C_4$-Alkoxy-$C_1$-$C_4$-alkyl, Phenyl, Phenyl-$C_1$-$C_4$-alkyl, $C_1$-$C_4$-Alkylcarbonyl, $C_1$-$C_4$-Alkoxycarbonyl, Cyano,

$Het^2$ 2-Pyridyl, 2-Chinolyl, 2-Pyrimidinyl bedeutet, wobei das heterocyclische Ringsystem gegebenenfalls substituiert ist durch Halogen, $C_1$-$C_8$-Alkyl, $C_3$-$C_6$-Cycloalkyl, $C_1$-$C_2$-Halogenalkyl, $C_1$-$C_4$-Alkoxy, $C_1$-$C_4$-Alkoxy-$C_1$-$C_4$-alkyl, Phenyl, Phenyl-$C_1$-$C_4$-alkyl, $C_1$-$C_4$-Alkylcarbonyl, $C_1$-$C_4$-Alkoxycarbonyl, Cyano,

sowie ihre pflanzenverträglichen Säureadditionssalze, Metallkomplexverbindungen und ihre N-Oxide.

**2.** Verfahren zur Herstellung von substituierten Oximether-Derivaten gemäß Anspruch 1, dadurch gekennzeichnet, daß man eine heterocyclische Verbindung der allgemeinen Formel II,

$Het^1$-O-H oder $Het^2$-S-H (II)

in der $Het^1$ und $Het^2$ die in Anspruch 1 genannten Bedeutungen haben, mit einem Benzylhalogenid der allgemeinen Formel III umsetzt,

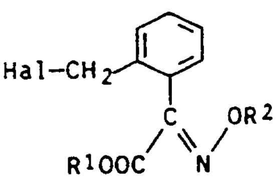 (III)

in der Hal für Brom, Chlor oder Jod steht und in der $R^1$ und $R^2$ die obengenannten Bedeutungen hat.

3. Verfahren zur Bekämpfung von Pilzen, dadurch gekennzeichnet, daß man die Pilze oder die von pilzbefall bedrohten Materialien, Pflanzen, Saatgüter oder den Erdboden mit einer fungizid wirksamen Menge einer Verbindung gemaäß Anspruch 1 behandelt.

4. Fungizid, enthaltend einen inerten Trägerstoff und eine fungizid wirksame Menge einer Verbindung gemäß Anspruch 1.

5. Verfahren zur Herstellung eines fungiziden Mittels, dadurch gekennzeichnet, daß man eine oder mehrere Verbindungen gemäß Anspruch 1 mit einem festen oder flüssigen Trägerstoff sowie gegebenenfalls mit einem oder mehreren oberflächenaktiven Mitteln mischt.

6. Verbindung der Formel Ia gemäß Anspruch 1, dadurch gekennzeichnet, daß $R^1$ und $R^2$ Methyl, $Het^1$ 6-Methyl-2-pyridyl bedeutet.

7. Verbindung der Formel Ib gemäß Anspruch 1, dadurch gekennzeichnet, daß $R^1$ und $R^2$ Methyl, $Het^2$ 6-Ethyl-2-pyridyl bedeutet.

8. Verbindung der Formel Ia gemäß Anspruch 1, dadurch gekennzeichnet, daß $R^1$ und $R^2$ Methyl, $Het^1$ 6-Isopropyl-2-pyridyl bedeutet.

9. Verbindung der Formel Ib gemäß Anspruch 1, dadurch gekennzeichnet, daß $R^1$ und $R^2$ Methyl, $Het^2$ 6-tert.Butyl-2-pyridyl bedeutet.

**Claims**

1. An oxime ether derivative of the formulae Ia and Ib

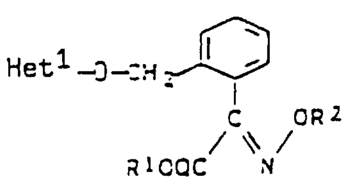 Ia 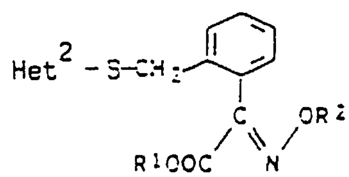 Ib

where
$R^1$ and $R^2$ are each hydrogen or $C_1$-$C_5$-alkyl,
$Het^1$ is pyridyl, quinolyl, pyrimidinyl, pyrazinyl, pyridazinyl, thienyl, quinoxalinyl, isoxazolyl, benzoxazolyl or benzothiazolyl, the heterocyclic ring system being unsubstituted or substituted by halogen, $C_1$-$C_8$-alkyl, $C_3$-$C_6$-cycloalkyl, $C_1$-$C_2$-haloalkyl, $C_1$-$C_4$-alkoxcy, $C_1$-$C_4$-alkoxy-$C_1$-$C_4$-alkyl, phenyl, phenyl-$C_1$-$C_4$-alkyl, $C_1$-$C_4$-alkylcarbonyl, $C_1$-$C_4$-alkoxycarbonyl or cyano, and
$Het^2$ is 2-pyridyl, 2-quinolyl or 2-pyrimidinyl, the heterocyclic ring system being unsubstituted or substituted by halogen, $C_1$-$C_8$-alkyl, $C_3$-$C_6$-cycloalkyl, $C_1$-$C_2$-haloalkyl, $C_1$-$C_4$-alkoxy, $C_1$-$C_4$-alkoxy-$C_1$-$C_4$-alkyl, phenyl, phenyl-$C_1$-$C_4$-alkyl, $C_1$-$C_4$-alkylcarbonyl, $C_1$-$C_4$-alkoxycarbonyl or cyano,
and their plant-tolerated acid addition salts, metal complexes and N-oxides.

2. A process for the preparation of a substituted oxime ether derivative as claimed in claim 1, wherein a heterocyclic compound of the formula II

$Het^1$-O-H or $Het^2$-S-H (II)

where $Het^1$ and $Het^2$ have the meanings stated in claim 1, is reacted with a benzyl halide of the formula III

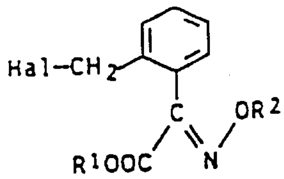

(III)

where Hal is bromine, chlorine or iodine and $R^1$ and $R^2$ have the abovementioned meanings.

3. A method for controlling fungi, wherein the fungi, or the materials, plants, seed or the soil threatened by fungus attack are treated with a fungicidally effective amount of a compound as claimed in claim 1.

4. A fungicide containing an inert carrier and a fungicidally effective amount of a compound as claimed in claim 1.

5. A process for the preparation of a fungicide, wherein one or more compounds as claimed in claim 1 are mixed with a solid or liquid carrier and, if required, with one or more surfactants.

6. A compound of the formula Ia as claimed in claim 1, wherein $R^1$ and $R^2$ are each methyl and $Het^1$ is 6-methyl-2-pyridyl.

7. A compound of the formula Ib as claimed in claim 1, wherein $R^1$ and $R^2$ are each methyl and $Het^2$ is 6-ethyl-2-pyridyl.

8. A compound of the formula Ia as claimed in claim 1, wherein $R^1$ and $R^2$ are each methyl and $Het^1$ is 6-isopropyl-2-pyridyl.

9. A compound of the formula Ib as claimed in claim 1, wherein $R^1$ and $R^2$ are each methyl and $Het^2$ is 6-tert-butyl-2-pyridyl.

**Revendications**

1. Dérivés d'oximéther des formules Ia et Ib

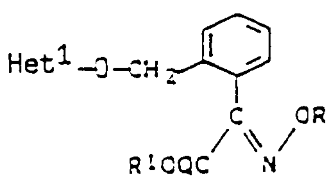

Ia

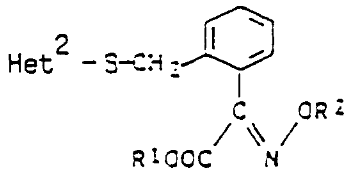

Ib

dans lesquelles
$R^1$ et $R^2$ représentent hydrogène ou alkyle en $C_1$-$C_5$
$Het^1$ représente pyridyle, quinolyle, pyrimidinyle, pyrazinyle, pyridazinyle, thiényle, quinoxalinyle, isoxazolyle, benzoxazolyle ou benzothiazolyle, le système annulaire hétérocyclique étant éventuellement substitué par halogène, alkyle en $C_1$-$C_8$,cycloalkyle en $C_3$-$C_6$, halogénalkyle en $C_1$-$C_2$, alcoxy en $C_1$-$C_4$, alcoxy en $C_1$-$C_4$-alkyle en $C_1$-$C_4$, phényle, phényle-alkyle en $C_1$-$C_4$, (alkyle en $C_1$-$C_4$)-carbonyle, (alcoxy en $C_1$-$C_4$)-carbonyle, cyano,
$Het^2$ représente 2-pyridyle, 2-quinolyle, 2-pyrimidinyle, le système annulaire hétérocyclique étant éventuellement substitué par halogène, alkyle en $C_1$-$C_8$, cycloalkyle en $C_3$-$C_6$, halogénoalkyle en $C_1$-$C_2$, alcoxy en $C_1$-$C_4$, alcoxy en $C_1$-$C_4$-alkyle en $C_1$-$C_4$, phényle, phényle-alkyle en $C_1$-$C_4$, (alkyle en $C_1$-$C_4$)-carbonyle, (alcoxy en $C_1$-$C_4$)-carbonyle, cyano,
ainsi que leurs sels d'addition d'acide, composés complexes métalliques et leur N-oxydes, acceptable pour les plantes.

2. Procédé de préparation de dérivés d'oximéther substitué selon la revendication 1, caractérisé par le fait que l'on fait réagir un composé hétérocyclique de la formule générale II

$Het^1$-O-H ou $Het^2$-S-H (II)

dans laquelle $Het^1$ et $Het^2$ ont les significations indiquées dans la revendication 1 avec un halogénure de benzyle de la formule générale III

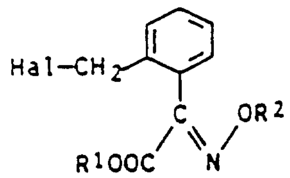

(III)

dans laquelle Hal est mis pour brome, chlore ou iode et où $R^1$ et $R^2$ ont les significations sus-indiqués.

**3.** Procédé de lutte contre les champignons, caractérisé par le fait que l'on traite les champignons ou les matériaux, plantes ou le sol menacés d'une attaque par champignons, avec une quantité efficace au point de vue fongicide d'un composé selon la revendication 1.

**4.** Fongicide, contenant un support inerte et une quantité efficace au point de vue fongicide d'un composé selon la revendication 1.

**5.** Procédé de préparation d'un fongicide, caractérisé par le fait que l'on mélange un ou plusieurs composés selon la revendication 1 avec un support solide ou liquide ainsi qu'avec éventuellement un ou plusieurs agents tensio-actifs.

**6.** Composé de formule Ia selon la revendication 1, caractérisé par le fait que $R^1$ et $R^2$ représente méthyle, $Het^1$, 6-méthyl-2-pyridyle.

**7.** Composé de formule Ib selon la revendication 1, caractérisé par le fait que $R^1$ et $R^2$ représente méthyle, $Het^2$, 6-éthyl-2-pyridyle.

**8.** Composé de formule Ia selon la revendication 1, caractérisé par le fait que $R^1$ et $R^2$ représente méthyle, $Het^1$, 6-isopropyl-2-pyridyle.

**9.** Composé de formule Ib selon la revendication 1, caractérisé par le fait que $R^1$ et $R^2$ représente méthyle, $Het^2$, 6-tert.butyl-2-pyridyle.